# EUROPEAN PATENT APPLICATION

(11) **EP 4 450 483 A1**
(43) Date of publication of application: **23.10.2024**
(21) Application number: 23168162.8
(22) Date of filing: 17.04.2023
(51) Int. Cl.: C07C 29/151, C10B 53/00, C10B 53/02, C10B 53/07, C10J 3/00, C10K 1/02, C10K 1/32, C10K 3/04, B01D 53/14

(54) **METHOD AND SYSTEM FOR FLEXIBLE PRODUCTION OF HYDROGEN AND HYDROCARBONS FROM PRODUCT GAS**

(71) Applicant: PAUL SCHERRER INSTITUT, 5232 Villigen PSI (CH)
(72) Inventor: Schildhauer, Tilman J., 5200 Brugg (CH)
(74) Representative: Fischer, Michael

(57) **Abstract**

It is desirable to design a plant such that it is flexible with respect to the product spectrum and to flexible integration of hydrogen as fuel besides biomass, while minimizing the capital costs by e.g. multipurpose reactors. Even on the feedstock side, some flexibility could be useful, i.e. using besides hydrogen also CO₂ as feed for the production of storable energy carriers, e.g. hydrocarbons, while using the stored energy carrier at least as partial feedstock in times when hydrogen production is desired.

This objective is achieved according to the present invention by a method/system for producing hydrogen and/or methanol, comprising :
a) thermochemically converting in terms of gasification and/or pyrolysis a carbonaceous feedstock from biomass or wastes yielding a product gas that comprises at least CO, H₂ and optionally unsaturated hydrocarbons, such as ethylene, acetylene, benzene;
b) optionally providing a gas cleaning section to remove catalyst poisons, e.g. Sulphur species, from the product gas;
c) bringing the product gas into a fluidized bed reactor and reacting the product gas in the presence of a catalyst and/or a sorbent based on e.g. copper, molybdenum and/or iron that supports the conversion of CO and H₂O to hydrogen and CO₂ or to methanol depending on the operation conditions that are controllable in terms of the catalyst and/or sorbents used and/or the pressure and/or the temperature and/or the heat exchange in the fluidized bed reactor;
d) guiding the reacted product gas to an upgrading section that comprises a particle filter, an optional condensation step and at least one separation unit, such as a membrane, a scrubber, an absorption, a pressure swing adsorption or a temperature swing adsorption, a chemisorption, a liquefaction or combinations thereof, thereby separating a hydrogenrich stream or a methanol-rich stream from the effluent of the fluidized bed reactor.

This method and this system thus allow to use one single fluidized bed reactor to flexibly decide for the production of hydrogen or methanol thereby enabling a production that for example is enabled to reflect the availability of green hydrogen and/or renewable energy.

## Description

The present invention relates to a method and a system for the production of hydrogen and other energy carriers according to the needs of markets and the energy systems, as well as to the provision of CO2 rich streams for sequestration and negative emissions become more and more important in the efforts of de-carbonization.

The production of hydrogen from solid carbonaceous feedstock such as wood, wood processing residues, scrap wood, (municipal) solid waste, plastic wastes, spent tires, torrefied biomass, landscaping residues and similar can be achieved by thermal processes with under-stoichiometric or no addition of air or oxygen, e.g. gasification or pyrolysis type processes. While large scale entrained flow gasifiers (>100 MW) produce a gas comprising mainly hydrogen (H2), carbon monoxide (CO), steam and carbon dioxide (CO₂), the product gas of small and middle scale gasifiers (e.g. countercurrent fixed beds, bubbling fluidized beds, dual fluidized beds) usually contains also methane, ethylene, ethane, acetylene and/or aromatic compounds. Further, depending on the feedstock, significant amounts of sulfur species have to be expected.

To produce pure hydrogen, many processes suggest a conversion of methane by a reforming step to carbon oxides and hydrogen and at least one subsequent water gas shift step to convert steam and CO to further hydrogen and CO₂. The CO₂ is separated from the hydrogen, e.g. by an amine scrubber, and is then available for sequestration or even negative CO₂ emissions in case of biogenic feedstock.

The catalysts for methane conversion are very sensitive to sulfur poisoning and coke formation; this asks for upstream reforming/removal of aromatics and unsaturated hydrocarbons and deep desulfurization. In consequence, the temperature along the process chain is changing from > 800°C in the gasification and tar reforming section down to < 350°C for the gas cleaning and deep desulfurization, and then is increasing back to 900°C for the methane reforming before the water gas shift at <350°C. This adds significantly to the process complexity and its capital costs and limits the efficiency.

Due to the seasonal imbalance of the supply of renewable energy (photovoltaic systems more in summer, wind more in winter), the price of electricity varies daily and over the year, which also has an impact on the availability and price of renewable hydrogen (green hydrogen) based on water electrolysis. Therefore, plants for the conversion of chemical energy carriers should allow for flexibility in the product composition to adapt to the needs of the energy system along the seasonal variation. This could comprise especially to produce more hydrogen, e.g. for transport and mobility, when electricity is scarce and expensive; and to produce storable energy carriers such as methane and/or methanol, maybe even by integrating available renewable hydrogen (Power-to-X), when there is a surplus of renewable electricity.

Therefore, it is desirable to design a plant such that it is flexible with respect to the product spectrum and to flexible integration of hydrogen as fuel besides biomass, while minimizing the capital costs by e.g. multipurpose reactors. Even on the feedstock side, some flexibility could be useful, i.e. using besides hydrogen also CO₂ as feed for the production of storable energy carriers, e.g. hydrocarbons, while using the stored energy carrier at least as partial feedstock in times when hydrogen production is desired.

This objective is achieved according to the present invention by a method for producing hydrogen and/or methanol, comprising the steps of:
a) thermochemically converting in terms of gasification and/or pyrolysis a carbonaceous feedstock from biomass or wastes yielding a product gas that comprises at least CO, H₂ and optionally unsaturated hydrocarbons, such as ethylene, acetylene, benzene;
b) optionally providing a gas cleaning section to remove catalyst poisons, e.g. Sulphur species, from the product gas;
c) bringing the product gas into a fluidized bed reactor and reacting the product gas in the presence of a catalyst and/or a sorbent based on e.g. copper, molybdenum and/or iron that supports the conversion of CO and H₂O to hydrogen and CO₂ or to methanol depending on the operation conditions that are controllable in terms of the catalyst and/or sorbents used and/or the pressure and/or the temperature and/or the heat exchange in the fluidized bed reactor;
d) guiding the reacted product gas to an upgrading section that comprises a particle filter, an optional condensation step and at least one separation unit, such as a membrane, a scrubber, an absorption, a pressure swing adsorption or a temperature swing adsorption, a chemisorption, a liquefaction or a combinations thereof, thereby separating a hydrogen-rich stream or a methanol-rich stream from the effluent of the fluidized bed reactor.

With respect to the system, these objectives are achieved according to the present invention by a system for producing hydrogen and/or methanol, comprising:
a) a conversion stage enabled to thermochemically converting in terms of gasification and/or pyrolysis a carbonaceous feedstock from biomass or wastes yielding a product gas that comprises at least CO, H₂ and optionally unsaturated hydrocarbons, such as ethylene, acetylene, benzene;
b) optionally a gas cleaning section to remove catalyst poisons, e.g. Sulphur species, from the product gas;
c) a fluidized bed reactor enabled to react the product gas in the fluidized bed reactor in the presence of a catalyst and/or a sorbent based on e.g. copper, molybdenum and/or iron that supports the conversion of CO and H₂O to hydrogen and CO₂ or to methanol and water depending on the operation conditions that are controllable in terms of the catalyst and/or the sorbent used and/or the pressure and/or the temperature and/or the heat exchange in the fluidized bed reactor;
d) an upgrading section receiving the reacted product gas and comprising a particle filter, an optional condensation step and at least one separation unit, such as a membrane, a scrubber, an absorption, a pressure swing adsorption or a temperature swing adsorption, a chemisorption, a liquefaction or a combinations thereof, thereby separating a hydrogen-rich stream or a methanol-rich stream from the effluent of the fluidized bed reactor.

This method and this system thus allow to use one single fluidized bed reactor to flexibly decide for the production of hydrogen or methanol thereby enabling a production that for example is enabled to reflect the availability of green hydrogen and/or renewable energy.

Preferably, besides the hydrogen-rich stream or the methanol-rich stream, also a CO₂-rich stream can be separated.

Additionally, besides the hydrogen-rich stream or the methanol-rich stream and optionally the CO₂-rich stream, also a methane comprising stream can be produced that can be injected into a natural gas grid, or upgraded to Liquefied Natural Gas, or used in a combustion to produce heat, or used in a combined heat and power plant such as gas motor, (micro) gas turbine, high temperature fuel cells (SOFC, MCFC) .

In order to provide conditions to maintain the selectivity of the catalyst, a sorbent can at least partly remove CO₂ and/or Sulphur species from the product gas in the fluidized bed reactor to enhance the yield of hydrogen.

In a preferred embodiment of the present invention, connections and/or the operation conditions of the upgrading units downstream of the fluidized bed reactor can be adapted accordingly to either match with the generation of hydrogen or with the generation of methanol.

Additionally, an unreacted gas content downstream of the fluidized bed reactor can be guided into a methanation unit, optionally with subsequent injection into the gas grid or a liquefaction unit, or to a thermal plant such as combustion or combined heat and power plant.

Further, sorbents can be flexibly used to remove methanol and/or water with the goal to increase the methanol yield in the fluidized bed reactor.

Furthermore, hydrogen can be flexibly added to increase the conversion of carbon oxides. Additionally, CO₂ or CO₂-rich gas (e.g. biogas) can be added thereby partly or even fully replacing the product gas from a gasification/pyrolysis step.

Alternatively or also additionally, hydrocarbons, such as methanol, can be flexibly added to the product gas in the fluidized bed reactor or to replace the product gas.

In a further preferred embodiment of the present invention, the necessary heat, for example >250°C at low pressures, for the reforming of the hydrocarbons, such as methanol, can be provided from in-situ partial oxidation with small amounts of injected oxygen or can be supplied by immersed heat exchanger tubes heated by thermos-oil, hot water or boiling water/condensing steam wherein the thermo-oil or water in the heat exchanger tubes is heated up by an external heat sources or by combusting a methane and/or CO rich stream after hydrogen separation or by the off-heat of a methanation reactor to which the said gases are sent, optionally after at least partial hydrogen separation.

Preferably, the catalyst in the fluidized bed reactor can be operated predominantly in the bubbling fluidization regime or can be operated predominantly in the circulating fluidization regime.

Preferred embodiments of the present invention are provided in the attached dependent claims.

Preferred embodiments of the present invention are hereinafter explained in more detail with respect to the attached drawings that depict in:
- Figure 1: a first example for a system for the production of hydrogen and other energy carriers;

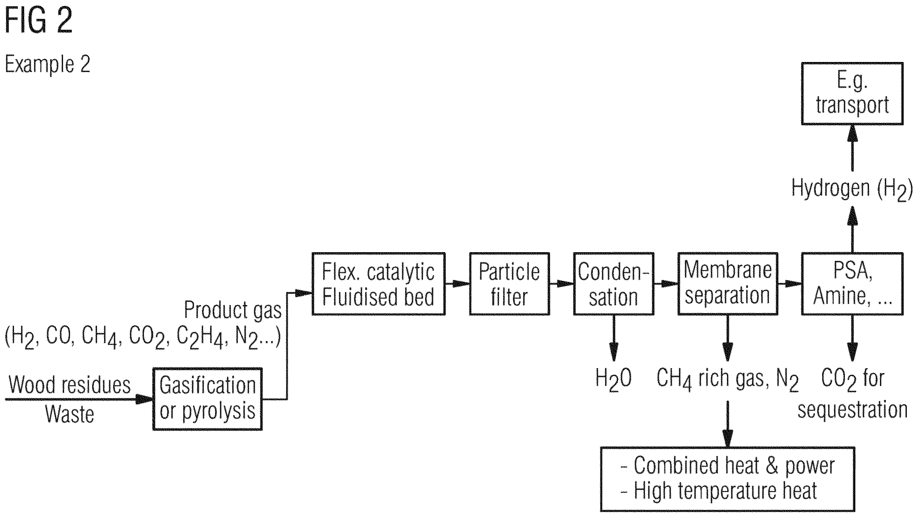

- Figure 2: a second example for a system for the production of hydrogen and other energy carriers;

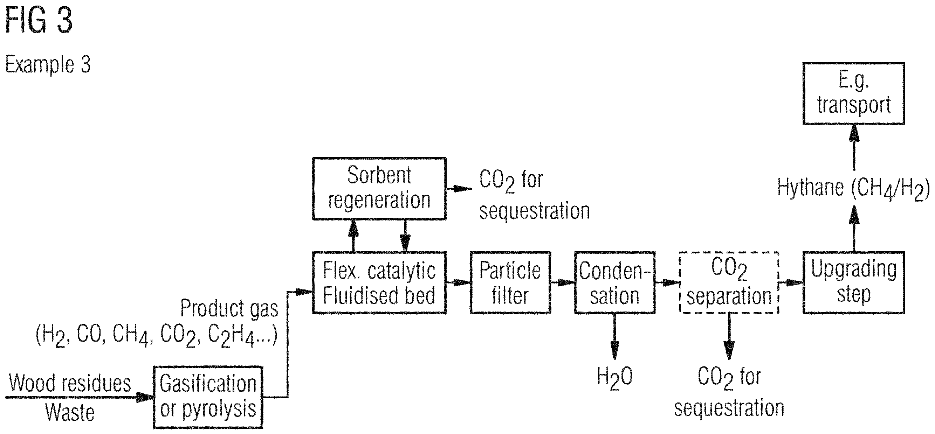

- Figure 3: a third example for a system for the production of hydrogen and other energy carriers;

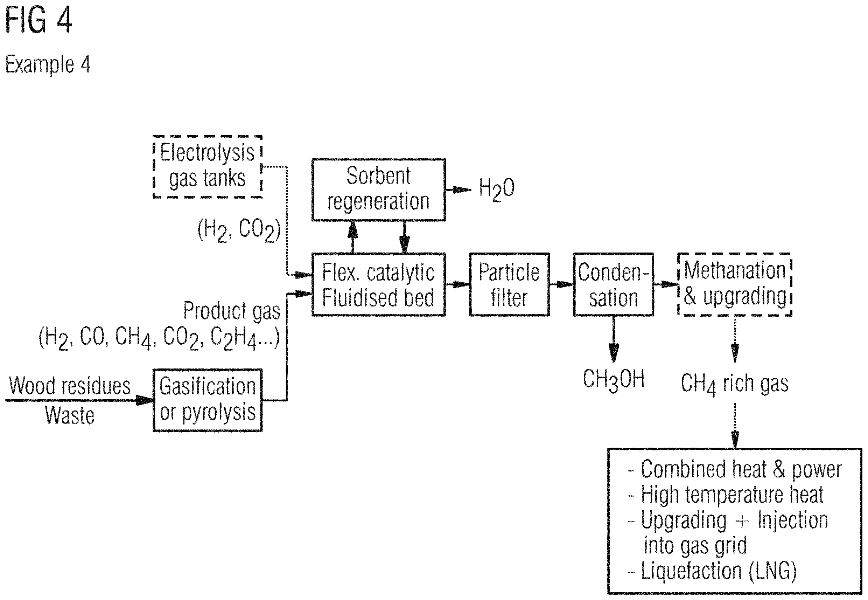

- Figure 4: a forth example for a system for the production of methanol and other energy carriers;

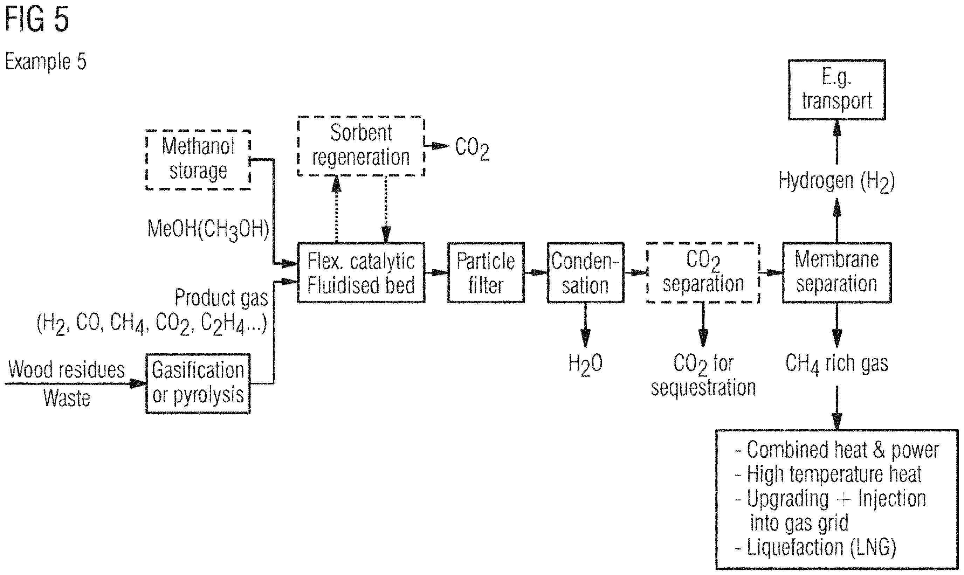

- Figure 5: a fifth example for a system for the production of hydrogen and other energy carriers.

### Concepts of the present invention

Producer gas from a gasification or pyrolysis comprising, besides at least CO and H₂, also methane and/or unsaturated hydrocarbon and/or aromatics is fed to a fluidized bed reactor filled with particles of a suitable catalyst for the conversion of CO and steam to H₂ and CO₂ (water gas shift reaction, WGS). The catalyst could be copper based, e.g. Cu/Zno/AlO₃, or MoS₂ based, especially in presence of sulfur species. The aim of the fluidization is to avoid or at least limit the deposition of carbon or coke on the catalyst due to the presence of impurities, such as aromatics and/or the unsaturated hydrocarbons. The movement of the particles by the fluidization causes their transport away from the entrance region, where the tendency to carbon/coke deposition is high due to high impurity concentrations. In the higher regions of the fluidized bed reactor, the impurity concentrations are lower due to their conversion. This allows the steam and hydrogen to react with carbon atoms on the catalyst surface, by which coke formation or carbon polymerization is diminished. This "internal regeneration by fluidisation" is therefore the key to conduct the water gas shift reaction in the presence of unsaturated hydrocarbons and/or aromatics. Using a state-of-the-art catalytic fixed bed reactors would require upstream conversion or removal of these species to avoid formation of coke or carbon deposits.

As many sources of the above-mentioned feedstocks are of limited capacity (30 MW or even smaller), the method suggested by the present invention converts most of the product gas to H₂, CO₂ and steam in the presence of unsaturated hydrocarbons (i.e. with simplified gas cleaning), while not converting the methane or only to low extent. The methane can be separated downstream from H₂ and CO₂, e.g. by means of membranes. As a result, most of the chemical energy is available as hydrogen while more than half of the carbon in the product gas is obtained as pure CO₂ available for sequestration (CCS) or further use (CCU), e.g. in PtX processes.

If needed, the internal regeneration could be supported by oxidative regeneration where the catalyst particles are contacted in the same or another vessel with a slightly oxidative atmosphere. Such oxidative treatment could also remove at least partially sulfur from the surface. It would be followed by reduction before or during the next catalytic half cycle.

A further advantage of the particle fluidization is the very high heat transfer to heat exchanger surfaces, which allows to keep the temperature close to isothermal conditions. As the WGS reaction is equilibrium limited and of significant exothermicity, the close to isothermal conditions allow maximum yield. With a state-of-the-art catalytic fixed bed reactor, avoiding of a hot spot and the connected equilibrium limitation would be difficult.

Another advantage of fluidized beds is that particles of different size or solid structures can be immersed in or transported through the fluidized bed of catalyst particles. If the additional particles or solid structures are able to adsorb CO₂, one of the reaction products of the WGS reaction, the equilibrium can be further shifted towards full conversion. Sorbent particles could either be smaller than catalyst particles, allowing the separation of catalyst particles and sorbent particles by entrainment and/or staged cyclones; or the sorbent particles could be larger than catalyst particles and would separate from the fluidized catalyst particles by gravity.

The reaction gas mixture leaving the reactor comprise the reaction products hydrogen and CO₂, some methane (that is not or hardly converted in the reactor) and might comprise unreacted aromatics, CO, steam and further hydrocarbons. To obtain pure hydrogen, suitable separation devices are used. These could be hydrogen selective permeable membranes (e.g. palladium based or similar), polymeric membranes that retain methane (e.g. from Biogas cleaning such as polyimide based or polypropylene based). Further or additionally, different separation methods could be applied (amine scrubber, pressurized water scrubber, pressure swing adsorption PSA, temperature swing adsorption TSA or combinations thereof), that help to separate species that are not, or not fully, separated by the membranes.

Besides hydrogen, also a methane or hydrocarbon rich gas stream is obtained that might be used as fuel, injected into the gas grid after suitable upgrading, or be used for thermal applications including industrial heat, heating of fermenters and buildings, or for electricity production in turbines or combined heat and power plants.

A further product from the process is a CO₂ rich stream that could be transported to a suitable sequestration site to enable negative emissions, or could be used for chemical processes, or after some storage for a PtX process.

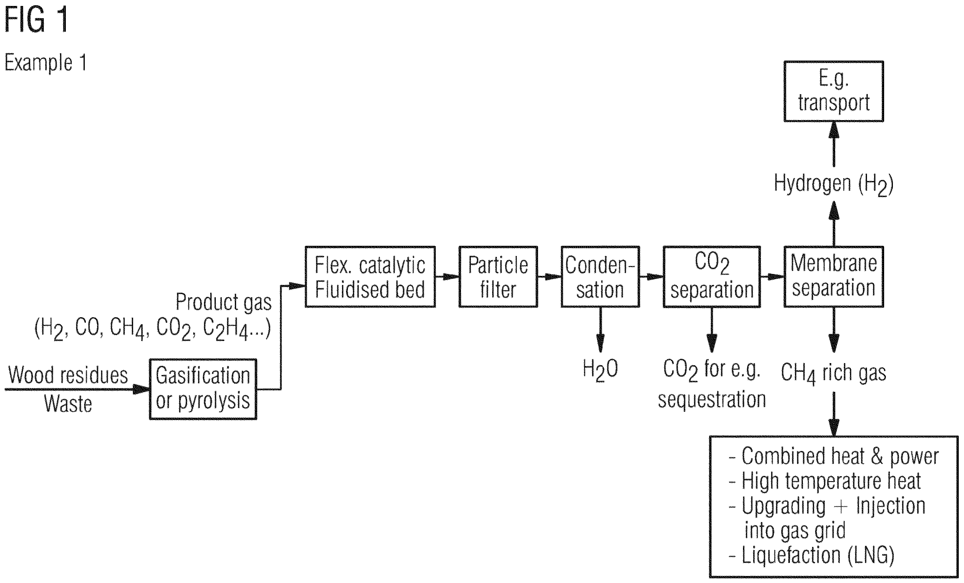

### Example 1 shown in Figure 1

A dual fluidized bed wood gasifier produces (downstream of its oil scrubber) a gas mixture of (dry) 40% H₂, 23% CO₂, 22% CO, 10% CH₄, 3%C₂H₄, 1% N₂, 1% aromatic compounds. Such a high level of unsaturated hydrocarbons would deactivate catalyst in standard fixed bed reactors by fast coke formation. After at least one desulfurization step (e.g. ZnO at >250°C), the gas is fed with additional steam to the fluidized bed water gas shift reactor which is the core of the present invention. CO and steam react to additional hydrogen and CO₂. Due to the excellent temperature control, high conversion of CO is possible. Methane in the feed is not converted. Ethylene (C₂H₄) is hydrogenated to ethane and partly converted to methane, similar as the aromatics.

After a condensation step, the hydrogen amount is increased by a factor 1.5, while the CO₂ content is doubled. The methane content is slightly increased but less than 1/3 of the CO₂ content. An amine scrubber now removes the CO₂, e.g. for sequestration, leaving behind a mixture of mainly hydrogen, methane and traces of ethane and nitrogen as well as humidity. A polymeric membrane unit (with maybe several steps and a recycle) now separates methane from hydrogen. This way relatively pure methane and hydrogen are produced that can be injected after optional final upgrading into the respective transport and distribution infrastructure (tanks, pipelines, liquefaction etc.). Alternatively, first the methane is separated as retentate, while a scrubber or a PSA separate hydrogen and CO₂ in the gas leaving the membrane separation as permeate.

### Example 2

Product gas from a fluidized bed gasifier or an updraft gasifier using air as gasifying medium is desulfurized. The product gas now comprises nearly 50% nitrogen, significant amounts of CO, hydrogen, but also methane and aromatics such as benzene. It is fed to the fluidized bed WGS reactor. Downstream the gas is filtered, water is separated by condensation, and hydrogen and CO₂ are separated by a membrane unit (with one or more steps and maybe a recycle), while methane and nitrogen stay in the retentate. The H₂/CO₂ rich permeate flow from the membrane unit is splitted (by e.g. a PSA or a scrubber) into a hydrogen rich stream and a CO₂ stream. The retentate stream containing methane and N₂ (from air blown gasification) is fed to a combustion, e.g. a CHP.

### Example 3

A product gas flow from a waste pyrolysis comprises hydrogen, CO, high amounts of sulfur (e.g. in case of spent tires or similar in the waste) as well as methane, and unsaturated hydrocarbons and/or aromatic compounds. After removal of high boiling tars in e.g. an oil scrubber, the product gas is fed to the fluidized bed WGS reactor where a molybdenum or MoS₂ based catalyst converts CO with steam in the presence of sulfur to produce additional hydrogen and CO₂ without or with significantly lower coke formation than to be expected in a fixed bed reactor. The conversion in the reactor might be supported by the addition of a suitable sorbent(s) such as CaO to capture CO₂ and maybe sulfur species. After a filter and a condensation step, CO₂ is separated by appropriate steps, while hydrogen and methane are together upgraded (e.g. dried) for further use as *"*hythane*"* or injected into the gas grids after suitable separation.

Further examples can be formed by other combinations of gas sources upstream, and separation steps downstream, of the fluidized bed WGS reactor with its internal regeneration to avoid or limit coke formation and carbon deposition. Also, different combinations of using the product gas streams rich in methane, hydrogen and/or CO₂ are possible.

### Example 4

The cooled fluidized bed reactor of example 1 is no longer operated under WGS conditions but uses now copper/zinc oxide based catalyst that is also well-known as methanol synthesis catalyst. This allows for product flexibility: by increasing the pressure and optionally slightly lowering the temperature in the fluidized bed, the equilibrium of the methanol synthesis is shifted to the product side, i.e. methanol is produced as co-product and can be separated by condensation. Such a reactor could also integrate external hydrogen, i.e. from electrolysis to increase the methanol yield. The yield of this valuable chemical can even be increased by applying sorption materials as it is shown in the European Patent Application 21 162 811, such that methanol becomes the main product. If available, the same parts of infrastructure (e.g. cyclones, regeneration reactor, standpipes, loop seals etc.) used for continuous sorbent addition to remove CO₂ (cf. to example 3) now can be used for the continuous addition and removal of sorbents to remove water and/or methanol.

As in the case of water gas shift reaction for hydrogen production, the heat of reaction can be removed by the thermos-oil system with its cooling coils/tubes immersed into the catalyst bed.

Further, such a reactor (with or without sorbent addition) could also integrate external CO₂ to augment or even substitute the fed product gas from gasification or pyrolysis. In times where hydrogen as product is of low interest, leftover hydrogen and carbon oxides could be sent, optionally after suited separation, to an optional methanation reactor downstream to increase the yield of methane that is another storable energy carrier. Alternatively, thermal use in SOFCs, MCFCs, gas motors, gas turbines, boilers or combustion systems is possible.

In case, hydrogen as product is not of interest anymore, the fluidized bed reactor could be converted without mechanical changes to a methanation system producing renewable methane. This could be done by exchanging the catalyst to e.g. a Nickel-based catalyst and optional adaptation of operation conditions (e.g. pressure and temperature) within the reactor and the downstream upgrading steps as well as the connections between the latter. Alternatively, the fluidized bed reactor could be bypassed to convert all gas in the optional methanation reactor mentioned in the paragraph above.

### Example 5

The cooled fluidized bed reactor with a suited catalyst, e.g. a CuO/ZnO based one, could also be used to re-convert the stored methanol to a hydrogen rich gas. The necessary heat (>250°C at low pressures) could come from in-situ partial oxidation with small amounts of injected oxygen or be supplied by the thermo-oil system. The thermo-oil could be heated up by external heat sources or by combusting the methane and/or CO rich gas after hydrogen separation or by the off heat of a methanation reactor to which the said gases are sent, optionally after at least partial hydrogen separation. The hydrogen production from methanol could augment or even substitute the conversion of gasification derived product gas.

Thus, the reactor and process concept suggested here allows flexibility both on the product side (hydrogen, methane, methanol) and the feedstock side (product gas, stored methanol, H2, CO2 containing gas streams). It can therefore serve different needs of the markets and (local) energy system within one plant by changing only operation conditions (e.g. volumetric flow rates, pressure and temperature) within the reactor and the downstream upgrading steps as well as the connections between the latter (e.g. by different valve positions).

### Example 6

A flexible plant could be built with a multipurpose fluidized bed reactor, in which cleaned and desulphurised product gas from a biomass gasifier is converted to mainly hydrogen, some methane, and CO₂ for sequestration and negative emission (according to example 1) during times of the year, when renewable electricity is scarce and expensive and thus water electrolysis is not attractive. In times, when sufficient renewable hydrogen is available, the pressure in the fluidized bed reactor is increased to >20 bar to favor methanol synthesis on the applied copper zinc oxide catalyst. The methanol is condensed out together with the H2O-content, while unreacted CO2, hydrogen, CO and the methane are upgraded downstream in the optional methanation reactor, and CO2 is separated by the membrane, scrubber or PSA which is also needed for the hydrogen up-grading in the rest of the year. Some valves and suited control systems may support to adapt the connections between the upgrading units and their respective operation conditions.

### Example 7

In another configuration of a flexible plant, stored methanol could be converted with steam to produce pure hydrogen for transport and mobility in the fluidized bed reactor and by subsequent upgrading (membrane, scrubber or PSA to remove CO2, PSA etc.). The necessary heat could be provided by staggered addition of small amounts of oxygen into the fluidized bed reactor where it reacts with methanol and/or hydrogen. The heat of this at least partial oxidation reaction is well dissipated and distributed by the moving particles, such avoiding a hotspot and securing high hydrogen yields.

The separated CO2 can be stored or sold or given to the atmosphere. Burnable gases downstream of the CO2 separation and the hydrogen separation could be burned to raise the steam for the reaction and/or to heat the thermos-oil system in case the oxygen addition is not possible or not sufficient.

To produce the stored methanol, hydrogen from electrolysis and stored CO2 or CO2 rich gas could be converted to methanol in the same fluidized bed reactor as explained in Example 6, especially in times when sufficient renewable hydrogen is available even after supplying the mobility and transport demands. Such a flexible plant would allow uninterrupted hydrogen supply e.g. for a fuel cell vehicle fleet during the whole year, balancing times of scarce and expensive electricity.

Also, other example for flexible plants can be suggested by combining aspects from the different examples given above.

## Claims

1. A method for producing hydrogen and/or methanol, comprising the steps of:
a) thermochemically converting in terms of gasification and/or pyrolysis a carbonaceous feedstock from biomass or wastes yielding a product gas that comprises at least CO, H₂ and optionally unsaturated hydrocarbons, such as ethylene, acetylene, benzene;
b) optionally providing a gas cleaning section to remove catalyst poisons, e.g. Sulphur species, from the product gas;
c) bringing the product gas into a fluidized bed reactor and reacting the product gas in the presence of a catalyst and/or a sorbent based on e.g. copper, molybdenum and/or iron that supports the conversion of CO and H₂O to hydrogen and CO₂ or to methanol depending on the operation conditions that are controllable in terms of the catalyst and/or sorbents used and/or the pressure and/or the temperature and/or the heat exchange in the fluidized bed reactor;
d) guiding the reacted product gas to an upgrading section that comprises a particle filter, an optional condensation step and at least one separation unit, such as a membrane, a scrubber, an absorption, a pressure swing adsorption or a temperature swing adsorption, a chemisorption, a liquefaction or combinations thereof, thereby separating a hydrogen-rich stream or a methanol-rich stream from the effluent of the fluidized bed reactor.

2. The method according to claim 1, where besides the hydrogen-rich stream or the methanol-rich stream, also a CO₂-rich stream is separated.

3. The method according to claims 1 or 2, where besides the hydrogen-rich stream or the methanol-rich stream and optionally the CO₂-rich stream, also a methane comprising stream is produced that is injected into a natural gas grid, or upgraded to Liquefied Natural Gas, or used in a combustion to produce heat, or used in a combined heat and power plant such as gas motor, (micro) gas turbine, high temperature fuel cells (SOFC, MCFC).

4. The method according to claims 1 to 3, wherein a sorbent at least partly removes CO₂ and/or Sulphur species from the product gas in the fluidized bed reactor to enhance the yield of hydrogen.

5. The method according to any of the preceding claims, wherein connections and/or the operation conditions of the upgrading units downstream of the fluidized bed reactor are adapted accordingly to either match the generation of hydrogen or the generation of methanol.

6. The method according to claim 5, wherein an unreacted gas content downstream of the fluidized bed reactor is guided into a methanation unit, optionally with subsequent injection into the gas grid or a liquefaction unit, or to a thermal plant such as combustion or combined heat and power plant.

7. The method according to any of the precedent claims, wherein flexibly sorbents are used that remove methanol and/or water to increase the methanol yield in the fluidized bed reactor.

8. The method according to any of the preceding claims, wherein flexibly hydrogen is added to increase the conversion of carbon oxides.

9. The method according to claim 8, wherein CO₂ or CO₂-rich gas (e.g. biogas) is added thereby partly or even fully replacing the product gas from a gasification/pyrolysis step.

10. The method according to any of the preceding claims, wherein flexibly hydrocarbons, such as methanol, are added to the product gas in the fluidized bed reactor or replace the product gas.

11. The method according to claim 10, wherein the necessary heat, for example >250°C at low pressures, for the reforming of the hydrocarbons, such as methanol, is provided from in-situ partial oxidation with small amounts of injected oxygen or is supplied by immersed heat exchanger tubes heated by thermos-oil, hot water or boiling water/condensing steam wherein the thermo-oil or water in the heat exchanger tubes is heated up by an external heat sources or by combusting a methane and/or CO rich stream after hydrogen separation or by the off-heat of a methanation reactor to which the said gases are sent, optionally after at least partial hydrogen separation.

12. The method according to any of the preceding claims, wherein the catalyst in the fluidized bed reactor is operated predominantly in the bubbling fluidization regime.

13. The method according to any of the claims 1 to 11, wherein the catalyst in the fluidized bed reactor is operated predominantly in the circulating fluidization regime.

14. A system for producing hydrogen and/or methanol, comprising:
a) a conversion stage enabled to thermochemically converting in terms of gasification and/or pyrolysis a carbonaceous feedstock from biomass or wastes yielding a product gas that comprises at least CO, H₂ and optionally unsaturated hydrocarbons, such as ethylene, acetylene, benzene;
b) optionally a gas cleaning section to remove catalyst poisons, e.g. Sulphur species, from the product gas;
c) a fluidized bed reactor enabled to react the product gas in the fluidized bed reactor in the presence of a catalyst and/or a sorbent based on e.g. copper, molybdenum and/or iron that supports the conversion of CO and H₂O to hydrogen and CO₂ or to methanol depending on the operation conditions that are controllable in terms of the catalyst and/or sorbents used and/or the pressure and/or the temperature and/or the heat exchange in the fluidized bed reactor;
d) an upgrading section receiving the reacted product gas and comprising a particle filter, an optional condensation step and at least one separation unit, such as a membrane, a scrubber, an absorption, a pressure swing adsorption or a temperature swing adsorption, a chemisorption, a liquefaction or combinations thereof, thereby separating a hydrogen-rich stream or a methanol-rich stream from the effluent of the fluidized bed reactor.
